# EUROPEAN PATENT SPECIFICATION

(11) **EP 3 049 567 B1**
(45) Date of publication and mention of the grant of the patent: **14.08.2019**
(21) Application number: 14777120.8
(22) Date of filing: 23.09.2014
(51) Int. Cl.: D06M 11/42, D06M 11/83, D06M 15/05, D06M 15/333, D06M 15/59, D06M 15/693, D06M 23/08, A61L 15/28, D06M 13/00, D06M 101/06

(54) **CELLULOSE FIBRES**
CELLULOSEFASERN
FIBRES DE CELLULOSE

(30) Priority: 23.09.2013 GB 201316867
(43) Date of publication of application: 03.08.2016
(73) Proprietor: Speciality Fibres and Materials Limited, London EC2A 2RS (GB)
(72) Inventor: DUFFY, Andrea, Coventry West Midlands CV6 5RS (GB); KETTLEWELL, Graeme, Coventry West Midlands CV6 5RS (GB)
(74) Representative: Bailey, Jennifer Ann
(86) International application number: PCT/GB2014/052894
(87) International publication number: WO 2015/040435

(56) References cited:
- CN-A- 102 120 043
- CN-A- 102 776 594
- US-A1- 2007 003 603

## Description

The present invention relates to a method for producing cellulose fibres, in particular cellulose fibres impregnated with metal nanoparticles. The invention also relates to the fibres produced thereby, and materials comprising the fibres.

Fibres useful as components in advanced wound care dressings are known in the art, particularly fibres based on cellulose or cellulose derivatives such as carboxymethylcellulose (CMC), cellulose ethyl sulfonate (CES) and salts thereof. For example, the commercial dressing AQUACEL (RTM) (sold by ConvaTec Inc of Skillman, New jersey, USA) is based on a carboxymethyl cellulose. The commercial dressing DURAFIBER (RTM) (sold by Smith and Nephew of Hull, United Kingdom) is made from a blend of cellulose fibres (TENCEL (RTM)) and CES fibres.

Metals including silver, copper, zinc and mercury are known for their antimicrobial properties. In particular, the use of metallic silver as an antibacterial agent has been known for many years. In recent years, a renewed interest has developed in the use of metallic silver as an antimicrobial agent, especially in wound dressings. This is driven in part by the development of antibiotic resistant bacteria, such as MRSA. Antibiotic resistant bacteria pose a real problem to medical practitioners and patients alike, as commonly used antibiotics are becoming less effective. Metallic silver, which is known to release ionic silver into a wound (when in contact with wound exudate), is a broad spectrum antibiotic which has been proven to be effective against such resistant bacteria. Current research suggests that due to its mode of action, metallic silver does not allow for the development of bacterial resistance.

Wound dressings currently available on the market primarily contain silver in its ionic form i.e. as a salt or other compound. However, the antibacterial properties of these dressings can be short lived due to the solubility of the silver salts or compounds in the aqueous nature of the wound environment, leading to an almost instantaneous and total release from the dressing. The rapid release of ionic silver into a wound could potentially cause toxic effects in host cells as well as bacteria. Some silver salts can also irritate the skin surrounding a wound, and prolonged contact has been reported to cause localised argyria, a permanent grey-blue staining of the skin. Silver salts in general are very sensitive to light, and show rapid and extensive discolouration (turning brown or even black). When utilised in wound dressings, this phenomenon can be experienced in general use and during sterilisation (by gamma irradiation) of the dressings prior to use, leading to less than appealing visual characteristics.

Methods of introducing silver into fibres include the use of silver zeolites or silver particles immobilised onto inert carriers such as silica or titania. Whilst antimicrobial dressings containing these materials can be effective, the loading of silver is limited due to the weight of carrier that can be incorporated into the fibres. These types of dressings are typically relatively expensive, due to the nature and amount of silver compound needed.

EP 1318842 describes a CMC fibre wherein silver ions are chemically bound to the fibre, and the use of this fibre in wound dressings. The silver-containing CMC fibre is then blended with gelling fibres, such as alginate fibres, to produce absorbent wound dressings.

EP1465673 B1 describes an antimicrobial wound dressing containing silver prepared from a blend of absorbent fibres and non-absorbent, metallic silver coated fibres. The metallic coated fibres are typically polyamide based, e.g. nylon. These dressings have excellent antimicrobial properties but suffer from poor wet strength.

EP 1490543 B1 describes an antimicrobial yarn containing 0.2-1.5% w/w silver nanoparticles which is designed for use in washable items whereby the nanoparticles remain adhered to the fibres for more than 100 washes. The nanoparticles are formed using a reducing agent such as glucose or vitamin C.

CN102120043 A describes an absorbent cotton gauze containing chitosan and nanosilver. A nanosilver solution is formed from silver nitrate solution using excess sodium borohydride and subsequently applied to the cotton gauze. Sodium borohydride is toxic and can react with moisture to produce flammable gas (diborane and hydrogen).

CN102776594 discloses a method comprising soaking cellulose fibre powder with NaOH-solution, removing the aqueous solution and mixing the cellulose fibres with silver nitrate.

US 2007/003603 describes methods for preparing antimicrobial silver compositions comprising silver nanoparticles, wherein the nanoparticles are stabilized by the addition of polymers.

It is an object of the present invention to mitigate at least some of the problems described above.

According to a first aspect of the present invention there is provided a method of producing cellulose fibres impregnated with metal nanoparticles, the method comprising:
swelling cellulose fibres in an aqueous alkali solution;
removing the swollen cellulose fibres from the aqueous alkali solution;
mixing the swollen cellulose fibres with an aqueous solution of a metal salt and a polymer solution so as to impregnate the fibres with metal nanoparticles; and
   removing the swollen cellulose fibres impregnated with metal nanoparticles from the solution, wherein the aqueous alkali solution comprises Group I hydroxide and Group I carbonate and/or bicarbonate.

As used herein, the term "metal nanoparticles" means particles of elemental metal having an average (i.e. mean) diameter of no more than 100 nm.

The metal may be selected from silver, copper, zinc, selenium, gold, cobalt, nickel, zirconium, molybdenum, gallium or iron or any combination thereof. In some embodiments, the metal is silver.

In some embodiments, the cellulose fibres are regenerated cellulose fibres. In further embodiments, the cellulose fibres are lyocell fibres. Lyocell fibres are made from solvent spun cellulose. Lyocell fibres are commercially available under the brand name 'TENCEL' (RTM) from Lenzing AG, Austria.

The cellulose fibres may have a linear density of from 1 to 2 dtex, or from 1.2 to 1.7 dtex. In some embodiments, the cellulose fibres have a degree of polymerization of from 400 to 700, from 500 to 650 or from 550 to 600. The cellulose fibres may have a dry tenacity of from 20 to 50, from 25 to 45 or from 30 to 40 cN/tex. The cellulose fibres may have an elongation at break of from 8 to 20%, from 9 to 18% or from 10 to 16%.

In some embodiments, the weight ratio of aqueous alkali solution to cellulose fibres is from 20:1 to 1:1, from 15:1 to 5:1, or from 12.5:1 to 7.5:1.

In particular embodiments, the aqueous alkali solution comprises sodium hydroxide. In some embodiments, the amount of compound used to prepare the solution (e.g. NaOH) is no more than 4.0 moles, no more than 3.5 moles, 3.0 moles, no more than 2.5 moles or no more than 2.0 moles per 30g of cellulose fibres. In further embodiments, the amount of compound is at least 0.25 moles, at least 0.5 moles, at least 0.75 moles or at least 1.25 moles per 30g of cellulose fibres. Sodium hydroxide is considered to be especially beneficial for opening up the pores in the swelling step.

The aqueous alkali solution comprises a Group I hydroxide (e.g. NaOH) and (i) a Group I carbonate (e.g. Na₂CO₃ or K₂CO₃) and/or (ii) a Group I bicarbonate (e.g. NaHCO₃ or KHCO₃). In some embodiments the ratio of the number of moles of Group I hydroxide to the number of moles of Group I carbonate and/or bicarbonate is at from 75:25 to 25:75 or from 66:34 to 50:50. The use of Group I hydroxide and Group I carbonate/bicarbonate is considered to be especially beneficial; the hydroxide is very effective for swelling the cellulose fibres and presence of residual carbonate is considered to be useful for the subsequent reduction of the metal salt. Without being bound by theory, the inventors propose that the residual carbonate reacts with the metal salt to generate a carbonate intermediate which is then reduced to metal nanoparticles.

In one embodiment the aqueous alkali solution comprises sodium hydroxide and a Group I carbonate. In one such embodiment the ratio of moles of sodium hydroxide to moles of G I carbonate is from 75:25 to 50:50.

In one embodiment the aqueous alkali solution additionally comprises LiCl.

In one embodiment the aqueous alkali solution has a pH of greater than 8, greater than 9, greater than 10, greater than 11 or greater than 12.

During the swelling step, it is preferred that the cellulose fibres are completely submerged in the aqueous alkali solution.

The swelling can be effected at any temperature, depending on the degree of swelling required. It will be understood that the cellulose fibres may be incubated in the aqueous alkali solution at a temperature and for a period of time that is sufficient to achieve the degree of swelling required. In some embodiments, the aqueous alkali solution containing the cellulose fibres is incubated at a temperature of from 20 °C to 120 °C, from 60 °C to 100 °C or from 80 °C to 90 °C. The fibres may be allowed to swell in the aqueous alkali solution for a period of time of from 1 minute to several hours. In some embodiments, the fibres are allowed to swell in the aqueous alkali solution for a period of time of from 5 minutes to 75 minutes, from 15 minutes to 45 minutes or from 20 minutes to 40 minutes.

It will be appreciated by those skilled in the art that swelling may be effected by placing a vessel containing the cellulose fibres in the aqueous alkali solution in a water or oil bath, so that the desired temperature can be maintained.

Once the swelling is complete the swollen cellulose fibres are separated from the aqueous alkali solution. The fibres may be compressed or manually squeezed to remove excess aqueous alkali solution from the fibres.

The swelling step is particularly advantageous since it allows the metal to penetrate the fibre as a result of the open structure after swelling. This allows higher metal loading of the cellulose fibres, as well as a more even distribution of metal in and on the fibres. Without wishing to be bound by theory, the inventors propose that swelling of the cellulose fibre opens up internal pores in the microfibrillar structure of cellulose, enabling increased penetration of the metal into the fibre. This allows incorporation of the metal nanoparticles into both the inner pore surfaces and the external fibre surface.

In some embodiments, the method further comprises washing the swollen cellulose fibres after removal from the aqueous alkali solution, and prior to adding the fibres to the aqueous solution of a metal salt and the polymer solution. The fibres may be washed in water, preferably hot deionized water. The washing helps to remove any residual alkali solution from the fibres. After washing, the fibres may be compressed or manually squeezed to remove the liquid. Multiple washing and squeezing steps may be carried out.

The next stage of the method involves impregnating the internal and external surfaces of the swollen cellulose fibres with metal nanoparticles, which are generated in situ by the reduction of a water soluble metal salt in the presence of a polymer. The inventors believe that the metal cation (e.g. Ag⁺) is reduced to form the metal nanoparticle (e.g. Ag⁰) via a redox reaction with the hydroxyl groups on the cellulose fibre. The polymer is believed to stabilize the metal nanoparticle by complexation, effectively capping the particle to prevent agglomeration.

The presence of residual alkali solution (from the swelling step) on the cellulose fibres may affect the subsequent redox reaction. Silver nitrate is believed to react with residual carbonate to yield a silver carbonate intermediate, which is then reduced to silver. The reduction of silver carbonate is believed to proceed relatively slowly such that the resulting nanoparticles can be capped by the polymer before agglomeration takes place. Silver nitrate reacts with residual hydroxide to yield a silver oxide which is less desirable.

It will be appreciated that in the step of mixing the swollen cellulose fibres with an aqueous solution of a metal salt and a polymer solution, the components of the mixture may be added to each other in any order. In some embodiments, mixing the swollen cellulose fibres with an aqueous solution of a metal salt and a polymer solution comprises adding the swollen cellulose fibres to an aqueous solution of a metal salt, and then adding a polymer solution to the aqueous solution of a metal salt containing the swollen cellulose fibres. Alternatively, the aqueous solution of a metal salt and the polymer solution may be simultaneously added to the fibres. Combining the components together simultaneously may advantageously help to prevent the formation of metal oxides.

In some embodiments, the weight ratio of aqueous solution of metal salt to cellulose fibres is from 20:1 to 1:1, from 15:1 to 5:1 or from 12.5:1 to 7.5:1. In some embodiments, the molar ratio of the alkali to the metal salt is no more than 20:1, preferably from 10:1 to 1:1 or more preferably from 5:1 to 1:1. In some embodiments, the amount of metal salt used per 30g of cellulose fibres is no more than 3 moles, no more than 2 moles no more than 1 mole, no more than 0.5 moles or no more than 0.25 moles. The amount of metal salt used per 30g of cellulose fibres may be at least 0.01 moles, at least 0.02 moles, at least 0.05 moles or at least 0.5 moles.

In some embodiments, the metal salt may be a nitrate, an acetate, a carbonate, a bicarbonate, a sulphate or mixtures thereof. In some embodiments, the metal salt may be a nitrate, an acetate, a sulphate or mixtures thereof. In further embodiments, the metal salt is selected from silver nitrate, silver acetate or silver sulphate and mixtures thereof. In particular embodiments, the metal salt is silver nitrate.

The polymer may be a polyamide, a polyimide, polyethyleneimine, polyvinylalcohol, pectin, albumin (bovine serum albumin or egg albumin), gelatin, carrageenan, a gum (such as xanthan, guar, arabic, acacia), cellulose (e.g. hydroxyethylcellulose, hydroxypropylcellulose, methylcellulose, hydroxypropylmethylcellulose etc.), poly (N-vinylpyrrolidone), poly (N-vinylcaprolactone), or mixtures thereof. In particular embodiments, the polymer solution is poly (N-vinylpyrrolidone), also known as Povidone or Polyvidone. In one such embodiment the polymer is poly (N-vinylpyrrolidone) having an average molecular weight of from 8 to 360kg/mol, from 30 to 80kg/mol or from 40 to 60kg/mol. An average molecular weight of from 40 to 60kg/mol provides excellent properties in terms of reaction viscosity and overall performance.

The amount of polymer solution added should be such that the overall liquid to fibre weight ratio of the metal solution/fibre mixture is not significantly altered. Thus, in some embodiments, the molar ratio of the polymer to the metal salt (based on the polymer repeat unit) is no more than 20:1, no more than 10:1, no more than 5:1 or no more than 2:1.

In some embodiments, the method further comprises incubating the mixture of the metal solution (i.e. the aqueous solution of metal salt), the polymer solution and the swollen cellulose fibres. During the incubation, the swollen cellulose fibres become impregnated with the aqueous solution of metal salt, and metal nanoparticles are formed. In some instances, a small amount of the metal salt may react with residual alkali (remaining from the swelling step), forming metal oxide within the fibres. The remaining metal salt is reduced to metal nanoparticles. The metal nanoparticles are thus formed in situ within the cellulose fibres. In situ formation of the metal nanoparticles is advantageous because it helps the nanoparticles to be evenly distributed throughout the fibres. Through this process, the metal nanoparticles become adhered to both the internal surfaces and the external surfaces of the cellulose fibres. Ensuring that the nanoparticles become trapped within the fibre structure, through attachment to the internal surfaces, is particularly important since it improves the metal loading/concentration of the fibres.

In some embodiments, the swollen cellulose fibres are impregnated with up to 25% metal nanoparticles w/w, based on the weight of the fibre. In some embodiments, the swollen cellulose fibres are impregnated with at least 5%, at least 10%, at least 12%, at least 15%, at least 18%, at least 19%, at least 20%, at least 21%, at least 22%, at least 24%, at least 26%, or at least 28% w/w metal nanoparticles. In further embodiments, the swollen cellulose fibres are impregnated with more than 1.5%, more than 2%, more than 3%, more than 4%, more than 5% or more than 8% w/w metal nanoparticles. In further embodiments, the swollen cellulose fibres are impregnated with from 5 to 30% w/w, from 10 to 25% w/w, from 15 to 23% w/w or from 20 to 22% w/w metal nanoparticles.

The polymer is thought to promote reduction of the metal ions to elemental metal (thought to result from a redox reaction with the hydroxyl groups on the cellulose as described previously). Without wishing to be bound by theory, the polymer is also believed to function as stabilizer for the formed metal nanoparticles by forming a complex with the nanoparticles and protecting them from agglomeration within the fibre, which can lead to non-uniform distribution of the metal. This increases the overall stability of the nanoparticles and allows for controlled, sustained release of a low concentration of metal nanoparticles from the fibres over time. This property is particularly useful when the fibres are incorporated into wound dressings. It is also believed that the particle size of the metal nanoparticles is at least partly controlled by the amount of polymer used in the process, together with the chosen reaction conditions. The metal nanoparticles may be described as being "polymer-stabilized" (i.e. complexed with the polymer).

It will be understood that the use of a traditional reducing agent is not required. Traditional reducing agents may be, for example, sodium borohydride, hydrazine hydrate, hydroxylamine, (tri)sodium citrate, starch, mono- or disaccharides (such as glucose, fructose and lactose), absorbic acid, or combinations thereof. In some embodiments the process is carried out in the absence of sodium borohydride, hydrazine hydrate, hydroxylamine, (tri)sodium citrate, starch, mono- or disaccharides (such as glucose, fructose and lactose) and/or absorbic acid. In some embodiments the method is carried out in the absence of a traditional reducing agent.

It will be understood that the mixture may be incubated for a period of time which is sufficient for the formation of metal nanoparticles. The metal nanoparticles may form instantaneously upon the mixing of the swollen cellulose fibres, the alkali solution of metal salt and the polymer solution, in which case the incubation may be transient. However, in some embodiments, the mixture is incubated for a period of time of from 5 to 180 minutes, from 15 to 150 minutes, from 30 to 120 minutes or from 60 to 90 minutes.

The reaction may be allowed to proceed at elevated temperature (i.e. above ambient temperature). In some embodiments, incubation of the mixture of the metal solution (i.e. the aqueous solution of metal salt), the polymer solution and the swollen cellulose fibres is carried out at a temperature of from 20°C to 120°C, from 60°C to 100°C or from 80°C to 90°C. It will be appreciated that incubation may be carried out by placing a vessel containing the mixture in a water or oil bath, so that the desired temperature can be maintained. Alternatively, the incubation may be carried out on the bench top.

Once the reaction is complete, the cellulose fibres impregnated with metal nanoparticles are removed from the liquid. The fibres may then be compressed or manually squeezed to remove any liquid remaining within the fibres.

In some embodiments, the method further comprises washing the fibres in order to remove excess reactants and reaction by-products. The fibres may be washed using a solvent such as water, alcohol or acid, or a combination thereof. In further embodiments, the method comprises shrinking the fibres by washing the fibres in (i.e. immersing/soaking the fibres in, or contacting the fibres with) a solvent, thereby trapping the silver nanoparticles within the fibre structure. The solvent may be any organic solvent, for example ethanol, propanol or isopropanol, ketones (e.g. acetone), esters and ethers (e.g. ethyl acetate, THF) or amides (e.g. DMF).

In further embodiments, the fibres are subjected to one or more washing cycles, wherein each washing cycle comprises a first wash using an acid solution (preferably citric acid), a second wash using water, and a third wash using an organic solvent. The concentration of the citric acid may be no more than 2 molar, preferably less than 1 molar or less than 0.5 molar. The second wash may comprise two steps, a first step using warm water and a second step using cold water. The water is preferably deionized. The organic solvent used in the third wash may be an alcohol, such as ethanol (or a denatured ethanol such as TSDA or IMS) or isopropanol, or a low molecular mass carbonyl compound such as acetone.

In some embodiments, the method further comprises drying the metal nanoparticle-impregnated cellulose fibres. Drying may be achieved by solvent evaporation, for example at an elevated temperature in the region of 50°C to 100°C.

The present invention thus provides a relatively simple process for the manufacture of cellulose fibres impregnated with metal nanoparticles (also referred to as cellulose-metal nanoparticle composite fibres). The resulting fibres have good strength and long term stability. The method of the invention is also believed to enable high metal loading of the cellulose fibres. This is important since there must be a sufficient quantity of metal present to provide an antimicrobial effect once the metal-loaded cellulose fibres have been blended with other types of fibres.

In accordance with the second aspect of the present invention there is provided cellulose fibres impregnated with metal nanoparticles.

The metal nanoparticles may be present at concentrations of up to 25% w/w, based on the weight of the fibre. In some embodiments, the metal nanoparticles are present at concentrations of at least 5%, at least 10%, at least 15%, at least 18%, at least 19%, at least 20%, at least 21%, at least 22%, at least 24%, at least 26%, or at least 28% w/w. In further embodiments, the metal nanoparticles are present at concentration of more than 1.5%, more than 2%, more than 3%, more than 4%, more than 5% or more than 8% w/w. In further embodiments, the metal nanoparticles are present at concentrations of from 5 to 30% w/w, from 10 to 25% w/w, from 15 to 23% w/w or from 20 to 22% w/w.

The cellulose fibres have an external fibre surface and inner pore surfaces. In one embodiment the metal nanoparticles are located in both the inner pore surfaces and the external fibre surface, i.e. the nanoparticles are not only located on the outside of the fibres but also within the fibres.

The metal nanoparticles may be evenly distributed throughout the fibre structure. The metal nanoparticles may be present on substantially all of the surfaces of the fibres.

The metal nanoparticles may have particle size (i.e. diameter) of less than 100 nm, less than 80 nm, 70 nm, less than 60 nm, less than 50 nm, less than 40 nm less than 30 nm, or less than 20 nm. In some embodiments, the average (i.e. mean) size is from 2 nm to 50 nm, from 5 nm to 30 nm or from 10 nm to 25 nm. In further embodiments, the average particle size is approximately 20 nm. The process of the present invention is considered to provide nanoparticles of very small particle size.

In some embodiments, the cellulose fibres impregnated with metal nanoparticles are further impregnated with a metal oxide (for example, silver oxide if an aqueous solution of silver salt is used in the method). The metal oxide may be present at concentrations of no more than 10% w/w, no more than 8 % w/w, no more than 5% w/w, no more than 3% w/w or no more than 1% w/w based on the weight of the fibre. In some embodiments, the metal oxide is present at concentrations of at least 0.1 % w/w, at least 0.2% w/w, at least 0.6% w/w, at least 0.8% w/w, at least 1% w/w, at least 1.2% w/w, at least 1.5% w/w, or at least 2% w/w based on the weight of the fibre. It will be understood that the metal oxide is undesirable since it can affect the handling of the fibres and should be minimised where possible.

In some embodiments, the metal oxide is present at a concentration of no more than 20%, no more than 15%, no more than 10% or no more than 5% of the concentration of metal nanoparticles.

In some embodiments, the cellulose fibres impregnated with metal nanoparticles are further impregnated with metal carbonate (for example, silver carbonate if a group I carbonate is employed in the aqueous alkali solution and a silver salt as the aqueous solution of metal salt). The metal carbonate may be present at concentrations of no more than 8 % w/w, no more than 5% w/w, no more than 3% w/w or no more than 1% w/w based on the weight of the fibre. In some embodiments, the metal carbonate is present at concentrations of at least 0.1 % w/w, at least 0.2% w/w, at least 0.6% w/w, at least 0.8% w/w, at least 1% w/w, at least 1.2% w/w, at least 1.5% w/w, or at least 2% w/w based on the weight of the fibre.

In some embodiments, the metal carbonate is present at a concentration of no more than 20%, no more than 15%, no more than 10% or no more than 5% of the concentration of metal nanoparticles.

In some embodiments, the cellulose fibres impregnated with metal nanoparticles are further impregnated with a polymer. The polymer will be present in small quantities if the method of the first aspect is employed to produce the fibres. The polymer may be present in an amount up to 30% w/w. In some embodiments, the cellulose fibres contain polymer at a concentration of no more than 30%, no more than 25%, no more than 20%, no more than 15%, no more than 10% or no more than 5%. In some embodiments, the fibres contain polymer at a concentration of at least 0.1%, at least 0.5%, at least 1%, at least 2%, at least 4%, at least 5%, at least 10% or at least 15%.

The polymer may be a polyamide, a polyimide, a polyethyleneimine, a polyvinylalcohol, pectin, albumin (bovine serum albumin or egg albumin), gelatin, carrageenan, gums (such as xanthan, guar, arabic, acacia) or cellulose (e.g. hydroxyethylcellulose, hydroxypropylcellulose, methylcellulose, hydroxypropylmethylcellulose etc.), poly (N-vinylpyrrolidone), poly (N-vinylcaprolactone), or mixtures thereof. Animal derived polymers may be undesirable in certain circumstances so in one embodiment the polymer is selected from a polyamide, a polyimide, a polyethyleneimine, a polyvinylalcohol, pectin, carrageenan, gums (such as xanthan, guar, arabic, acacia) or cellulose (e.g. hydroxyethylcellulose, hydroxypropylcellulose, methylcellulose, hydroxypropylmethylcellulose etc.), poly (N-vinylpyrrolidone), poly (N-vinylcaprolactone), or mixtures thereof. In particular embodiments, the polymer is poly (N-vinylpyrrolidone).

In some embodiments, the metal release rate of the cellulose fibres impregnated with metal nanoparticles is at least 30 ppm per day, at least 40 ppm per day, at least 50 ppm per day or at least 55 ppm per day, per 0.5g fibre, as measured in accordance with the method described in Example 1.3. In some embodiments, the metal release rate is no more than 100 ppm/day, no more than 90 ppm/day, no more than 80 ppm/day, no more than 70 ppm/day or no more than 65ppm/day, per 0.5g fibre. In some embodiments, the release rate is maintained for at least 3 days, at least 5 days or at least 7 days.

In some embodiments, the cellulose fibres impregnated with metal nanoparticles have a linear density of at least 1.4, at least 1.5 or at least 1.6 dtex, as measured using standard techniques. In some embodiments, the cellulose fibres have a break load of at least 4 N, at least 4.4 N, at least 4.5 N or at least 4.6 N. In some embodiments, the cellulose fibres have a breaking tenacity of at least 24, at least 26 or at least 28 cN/tex. In further embodiments, the fibres have a strain at break of at least 8.50, at least 9.00, at least 9.25, at least 9.50 or at least 9.75 %.

The cellulose fibres impregnated with metal nanoparticles may have a pH of less than 7.00, less than 6.80, less 6.70 or less than 6.60, less than 6.50, less than 6.40 or less than 6.30. The fibres may have a pH of more than 5.80, 6.00, more than 6.20, more than 6.40 or more than 6.50.

The present invention further provides cellulose fibres impregnated with metal nanoparticles obtainable by a method according to the first aspect of the invention. The method of the first aspect allows incorporation of the metal nanoparticles into both the inner pore surfaces and the external fibre surface.

The cellulose fibres impregnated with metal nanoparticles, and blends thereof, can be formed into fabrics using standard techniques, such as carding and needling, which will be well-known to those skilled in the art. Such fabrics are particularly useful for the manufacture of absorbent articles, such as wound dressings.

According to the third aspect of the invention, there is provided an absorbent material comprising a blend of cellulose fibres impregnated with metal nanoparticles according to the second aspect of the invention with at least one other type of fibre.

The at least one other type of fibre may be a gelling and/or a non-gelling fibre. Examples of gelling fibres include fibres based on polysaccharides such as alginate (i.e. a salt of alginic acid), modified cellulose (for example CES, CMC), modified chitosan (e.g. carboxymethylchitosan, sulfonated chitosan, carboxyethyl chitosan) or mixtures thereof. Further examples of gelling fibres include polyacrylates or copolymers thereof, polyethyleneoxides and polyacrylamides. Examples of non-gelling fibres include fibres based on polyester, polyethylene, polypropylene or polyamide, cellulose (e.g. lyocell fibres such as TENCEL (RTM)), thermoplastic bicomponent fibres, and glass fibres.

In some embodiments, the material comprises cellulose fibres impregnated with metal nanoparticles blended with CES fibres and/or lyocell fibres and/or CMC (carboxymethyl cellulose) fibres. In some embodiments, the blend comprises at least 5%, at least 8%, at least 10% or at least 15% cellulose fibres impregnated with metal nanoparticles (w/w, based on the total weight of the fibres). In some embodiments, the blend comprises at least 5%, at least 10%, at least 15% or at least 20% lyocell fibres. In some embodiments, the blend comprises at least 10%, least 20%, least 40%, least 60% or least 80% CES fibres. In some embodiments, the blend comprises at least 10%, least 20%, least 40%, least 60% or at least 80% CMC fibres.

In some embodiments, the absorbent material comprises a blend of 5-30% w/w cellulose fibres impregnated with metal nanoparticles, 5-30% w/w lyocell fibres and 40-90% w/w CES fibres. In a further embodiment, the absorbent material comprises a blend of 10% cellulose fibres impregnated with metal nanoparticles (e.g. silver nanoparticles) with 10% lyocell fibres (e.g. TENCEL (RTM) fibres) and 80% CES fibres.

In some embodiments, the absorbent material comprises a blend of 2-20% w/w cellulose fibres impregnated with metal nanoparticles, 10-30% w/w lyocell fibres and 50-90% CMC fibres.

In some embodiments, the absorbent material comprises more than 0.2%, more than 0.5%, more than 0.8%, more than 1%, more than 1.2% or more than 1.5% metal w/w, based on the total weight of the blended fibres. The absorbent material may comprise less than 8%, less than 5% w/w, less than 3% w/w or less than 2% w/w metal.

In some embodiments, the material has an absorbency of more than 15, more than 16, more than 17, more than 18, more than 19, more than 20, more than 22 or more than 25 grams of liquid per gram of material.

In some embodiments, the absorbent material has a dry tensile strength in the machine direction (M direction) of from 6 to 12 N/cm. In some embodiments, the absorbent material has a wet tensile strength in the machine direction (M direction) of 1 to 2 N/cm. In some embodiments, the absorbent material has a dry tensile strength in the cross direction (X direction) of 25 to 35 N/cm. In some embodiments, the absorbent material has a wet tensile strength in the cross direction (X direction) of 2 to 6 N/cm.

It is preferred that the blend of fibres is selected so as to provide the desired metal content whilst retaining absorption and strength characteristics.

The blend of fibres may be woven or non-woven. In some embodiments, the fibres are non-woven.

The absorbent material may be used to make items such as textiles, clothing, furniture, antimicrobial reinforcing fibres, and wound dressings.

According to a fourth aspect of the present invention there is provided an absorbent article comprising the absorbent material of the third aspect.

In some embodiments, the absorbent article is a wound dressing. The wound dressing may be in the form of swabs, wound pads, wadding ribbons, sponges, nets and bandages. The absorbent material may form one of a plurality of layers of the wound dressing. The wound dressing may further comprise a perforated film which is applied to one or more surfaces of the dressing.

In some embodiments, the absorbent article is a surgical item such as a face mask, surgical gown or surgical drape.

The metal content of the absorbent article may be from 0.1 to 10%w/w, from 0.15 to 8%w/w, from 0.2 to 6%w/w, 0.5 to 5% w/w, from 1 to 3% w/w or from 1.5 to 2% w/w, based on the total weight of the blended fibres.

In some embodiments, the metal release rate of the absorbent material or the absorbent article is at least 3 ppm, at least 4 ppm, at least 5 ppm, at least 6 ppm, at least 7 ppm or at least 8 ppm metal per day per 0.5 g of article or material, as measured in accordance with the method of Example 3.3. In further embodiments, the release rate is no more than 20, no more than 16, no more than 12 than 10 ppm, no more than 9 ppm or no more than 8 ppm per day per 0.5 g material/article.

The absorbent material of the invention, and absorbent articles formed therefrom, are advantageous in that they provide a sustained, low level release of metal over a period of time. This property is particularly advantageous in that it allows for more extended wear of wound dressings, thereby increasing patient comfort by having less frequent dressing changes.

Without wishing to be bound by theory, it is thought that the use of a polymer in the preparation of the cellulose fibres improves the retention of the metal nanoparticles on the fibres, since the polymer is thought to be able to bind polar molecules such as cellulose.

Wound dressings comprising the cellulose fibres of the invention can be used to treat and/or prevent infection, and are particularly suitable for the management of chronic wounds. The absorbent material and absorbent articles of the invention also benefit from high wet and dry tensile strength, uniform and stable colour and high antibacterial efficiency.

Fabric made from the fibres of the invention is also relatively stable to light (or other radiation e.g. gamma) exposure, such that substantially no change in colour is observed when the material is exposed to radiation for a period of time.

The fibres of the invention, and materials and articles made therefrom, thus provide an alternative technology that does not rely on the delivery of metal salts into a wound environment, thereby reducing problems such as cytotoxicity or staining of the skin during use.

It will be understood that any of the above statements may apply equally to the first, second, third or fourth aspects of the invention, as appropriate.

Embodiments of the present invention will now be described by way of example and with reference to the accompanying Figures, in which:
Figure 1 is a UV spectrum of a sample of deionized water in which cellulose fibres made in accordance with an embodiment of the invention had been immersed;
Figure 2 is a UV spectrum of a sample of deionized water in which cellulose fibres made in accordance with another embodiment of the invention had been immersed;
Figure 3 is a histogram of count against particle size (µm) for nanoparticles made in accordance with an embodiment of the invention; and
Figure 4a is an SEM image of the exterior of a cellulose fibre impregnated with metal nanoparticles in accordance with an embodiment of the invention; Figures 4b and 4c are SEM images of cross-sections of fibres made in accordance with an embodiment of the invention.

### Example 1: Cellulose fibres impregnated with silver nanoparticles

### Reference example 1.1a Method of producing cellulose fibres impregnated with silver nanoparticles

The following describes a typical laboratory process for the preparation of cellulose fibres impregnated with silver nanoparticles. It will be appreciated that the quantities and conditions may be adjusted for scaling the process up or down.
1. 30g of dry TENCEL™ fibre (e.g. G600-862; 1.4 dtex, crimped) is placed into a glass vessel, such as a beaker or bottle.
2. 300 ml of aqueous sodium hydroxide solution (3 molar; prepared using freshly deionised water) is added to the vessel containing the fibres. All the fibres should be completely covered by the aqueous sodium hydroxide solution.
3. The vessel containing the fibres and sodium hydroxide solution is then sealed and placed into an oil or water bath, set at 90°C, to effect swelling of the fibres.
4. The fibres are allowed to swell in the aqueous sodium hydroxide solution for approximately 30 minutes.
5. Once the swelling is completed, the excess sodium hydroxide solution is decanted from the vessel and manually squeezed from the fibres.
6. The fibres are then rinsed in hot, freshly deionised water, using the same temperature/dwell conditions as used for the swelling stage, with subsequent manual squeezing to remove the liquid.
7. The fibres are then placed into a clean glass vessel and 100 ml aqueous silver nitrate solution (30% w/w; prepared using freshly deionised water) is added.
8. 200ml of a 15% solution of poly (N-vinylpyrrolidone) (ave. molecular weight of 40000 gmol⁻¹) in freshly deionised water is then added to the fibres/silver nitrate. It will be appreciated that the silver nitrate and polymer solutions may be added simultaneously to the fibres. The vessel containing the swollen cellulose fibres, the silver nitrate and the poly (N-vinylpyrrolidone) is then sealed and transferred back to the oil (or water) bath to effect the reduction of the silver nitrate, with subsequent in situ formation of poly (N-vinylpyrrolidone) stabilised silver nanoparticles. The temperature of the oil or water bath is set at 90°C.
9. The reaction is allowed to proceed for approximately 90 minutes.
10. Once the reaction is complete, the silvered fibres are removed from the reaction liquor and manually squeezed to remove the residual liquid from within the fibres.
11. The fibres are then subjected to a series of washing cycles in order to clean the fibres of excess reactants and reaction by products. The first wash utilises a weak citric acid solution (0.2 molar). The fibres are then washed with 300 ml warm deionised water, followed by a wash using cold deionised water. The fibres are then washed in acetone, as a means to 'de-swell' the fibres.
12. Once the fibres are clean and 'de-swollen' the solvent is removed by evaporation using elevated temperatures in the region of 50-100°C.

### Example 1.1b Modified method of producing cellulose fibres impregnated with silver nanoparticles

The method of 1.1a was repeated with the following modifications:
In step 2. an aqueous solution is prepared from 211g de-ionised water, 36g sodium hydroxide and 53g sodium carbonate (the ratio of the number of moles of sodium hydroxide to the number of moles of sodium carbonate is 1.8:1); and
In step 8. the poly (N-vinylpyrrolidone) has a molecular weight of 40 to 80kg/mol.

### 1.2 Physical properties of the cellulose fibres (method 1.1a)

Fibres produced according to the method 1.1a above were found to have a deep brown/black colour with soft drape and handle.

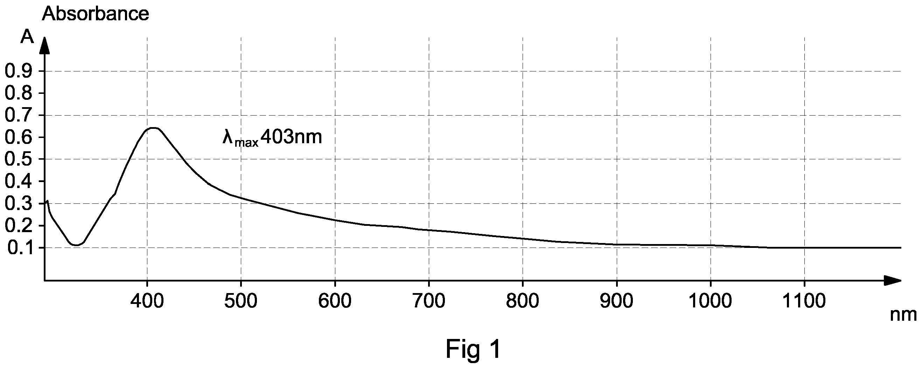

The fibres were immersed in a sample of deionized water, and the UV spectrum of the water recorded (Figure 1). A spherical nanoparticle having a mean particle size in the region of 20 nm absorbs UV light at approximately 402 nm. The spectrum therefore confirms that silver nanoparticles of approximately 20 nm in diameter were present in/on the fibres.

The fibres were also analysed using SEM-EDX. The image showed a uniform distribution of silver nanoparticles throughout the cellulose fibres.

Batches of fibres prepared according to the method above were found to have a silver content of 19.24%, 21.14%, 20.84% and 22.54% (measured by ICP-OES, includes all sources of silver i.e. silver nanoparticles and silver oxide).

The pH of the fibres was measured by placing 0.5g fibre in 50 ml deionized water, and the initial physical and mechanical properties of the fibres were measured. The results are shown in Table 1a, which also shows comparative data for TENCEL (RTM) and CES fibres. The tensile properties of the fibres were also measured after 6 months at ambient temperature. The results are shown in Table 1b.

**Table 1a**

| | **Initial Tensile Properties** | | | | **pH** |
|---|---|---|---|---|---|
| | **Linear Density, dtex** | **Break Load, N** | **Breaking Tenacity, cN/tex** | **Strain at Break %** | |
| **TENCEL (RTM)** | 1.49 | 4.96 | 33.48 | 10.82 | 7.12 |
| **CES** | 1.81 | 4.01 | 22.18 | 10.44 | 6.50 |
| **Cellulose impregnated with silver nanoparticles** | 1.64 | 4.65 | 28.37 | 9.75 | 6.57 |

The tensile properties of the silvered cellulose fibres were found to be comparable to those of other cellulose-based fibres. The linear density, break load and breaking tenacity of the silvered fibres were found to exceed that of CES. The pH of the silvered fibres was found to be similar to that of CES, but lower than that of TENCEL (RTM).

**Table 1b**

| | **Breaking Tenacity, cN/tex** | **Strain at Break %** |
|---|---|---|
| **TENCEL (RTM)** | 32.45 | 9.21 |
| **CES** | 23.39 | 9.26 |
| **Cellulose impregnated with silver nanoparticles** | 28.78 | 9.06 |

It was found that after 6 months the breaking tenacity and strain at break values of the silvered cellulose fibres were comparable to the values measured initially. The tensile properties of the fibres are therefore not degraded over time.

### 1.3 Release of metal from the cellulose fibres (method 1.1a)

0.5g of cellulose fibre impregnated with silver nanoparticles, made as described above, was placed into 50 ml deionized water and incubated at 37°C. Each subsequent day, 2 ml of the liquid was removed for analysis by UV-Vis. The liquid removed was replaced with fresh deionized water. The silver content of the liquid measured each day is shown in Table 2a.

**Table 2a**

| **Time (days)** | **Silver content (ppm)** |
|---|---|
| 1 | 61.4 |
| 2 | 62.5 |
| 3 | 59.6 |
| 4 | 57.8 |
| 5 | 60.1 |

The fibres are capable of providing a sustained release of silver over a period of time. This property is particular advantageous for wound dressings.

### 1.4 Antimicrobial properties of the cellulose fibres (method 1.1a)

0.5g of the cellulose fibres impregnated with silver nanoparticles, prepared as described above, was placed in 25g milk and incubated at 30°C for 7 days. The results are shown in Table 3.

**Table 3**

| **Fabric Sample** | **Silver (% w/w)** | **Antimicrobial Performance @ 37°C** |
|---|---|---|
| Blank Sample - No Fibre | NA | PS, O (very strong malodour), GAS |
| | | Observed after 2 days |
| Silvered Cellulose Fibre | 20.84 | NC |
| | | Observed after 7 days |

### Key

- *PS*: *Phase separation of milk*
- O: *Odour present*
- *GAS*: *Pressure build up in test vessel*
- *NC*: *No change*

The fibres impregnated with silver nanoparticles were therefore found to have effective antimicrobial properties, which were sustained over a period of time.

### 1.5 Physical properties of the hydroxide/carbonate produced cellulose fibres (method 1.1b)

Fibres produced according to the method 1.1b were found to have a deep brown colour with soft drape and handle. The fibres were even softer and easier to separate than those made using method 1.1a.

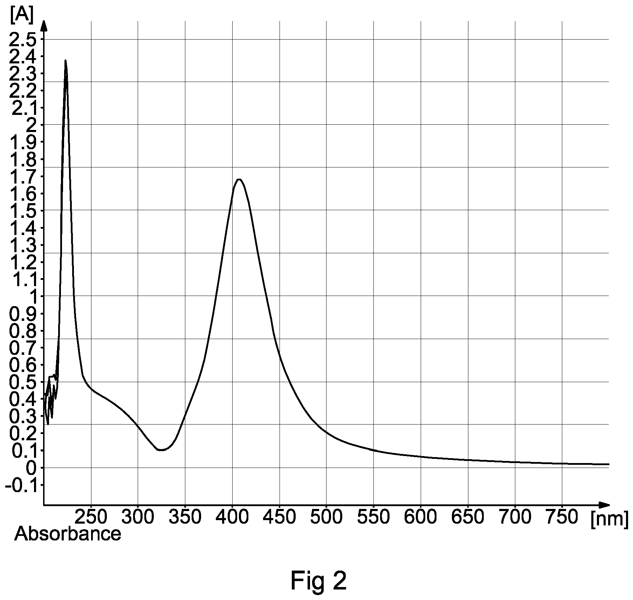

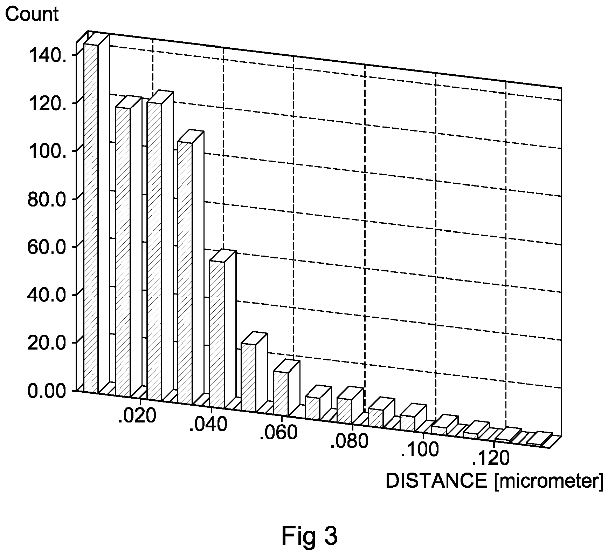

The fibres were immersed in a sample of deionized water, and the UV spectrum of the water recorded (Figure 2). A spherical nanoparticle having a mean particle size in the region of 30 nm absorbs UV light at approximately 405 nm. The spectrum therefore confirms that silver nanoparticles of approximately 30 nm in diameter were present in/on the fibres. TEM images of the nanoparticles in deionised water were also analysed to measure the particle size and the results are summarised in Figure 3, a histogram showing the distribution of silver nanoparticles (distance (µm) v. count). The histogram shows that the majority of the particles have a particle size in the region of 5 to 40 nm and is therefore consistent with the UV spectrum.

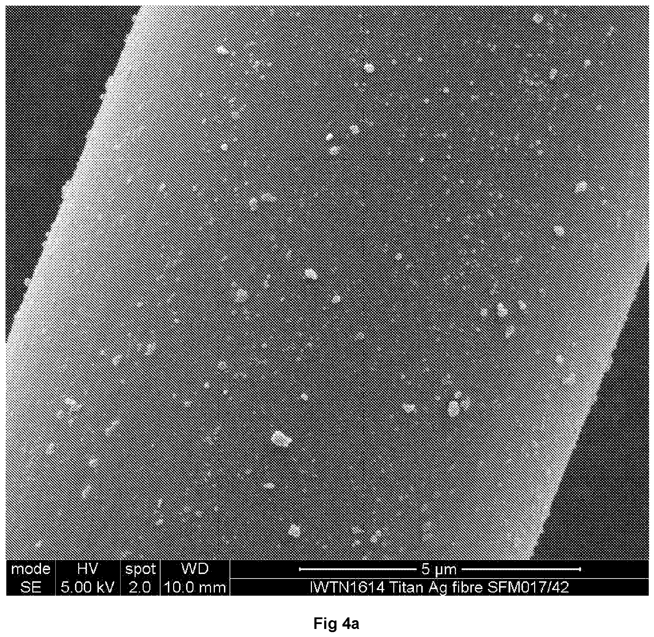

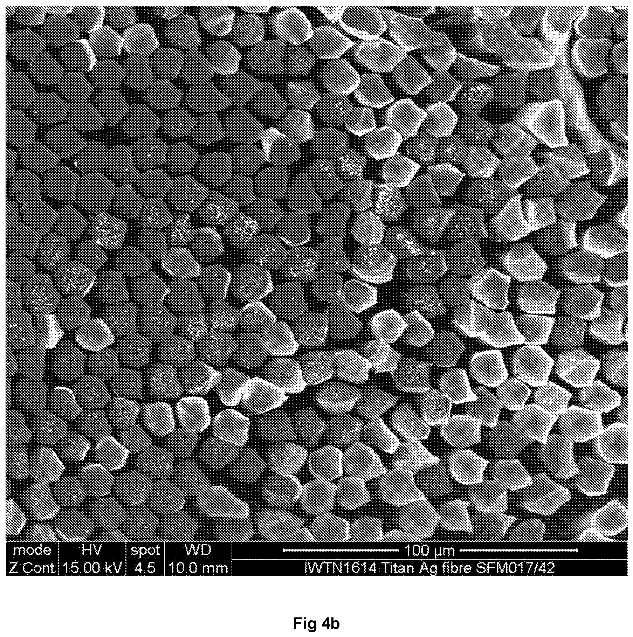

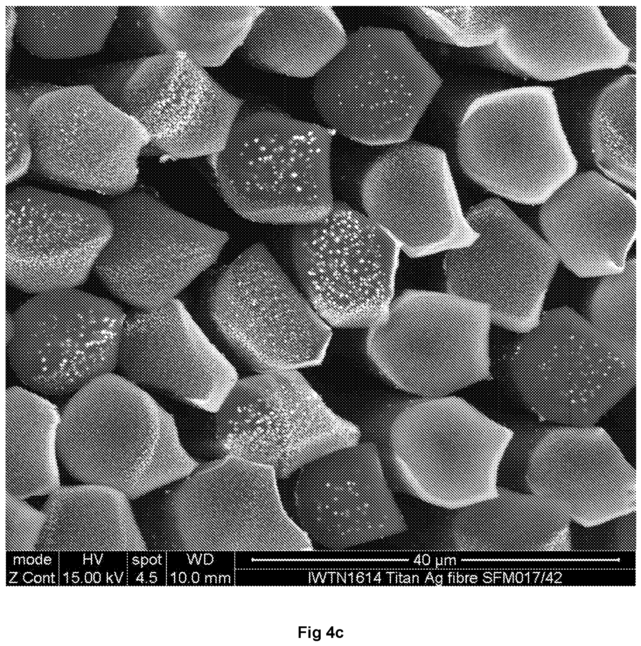

The fibres were also analysed using SEM-EDX (Figure 4). Figure 4a shows silver nanoparticles distributed across the exterior surface of a fibre. The backscattered SEM images 4 b and 4c show typical prepared cross-sections through representative specimens of the fibre sample. Backscattered imaging highlights the presence of any silver nanoparticles (high atomic number) which can be seen as higher contrast (bright) particles and/or concentrated regions of particles in the images. Bright particles can be seen within the fibres also on the exterior surface of the fibres. Some of the fibres are dark indicating an absence of silver nanoparticles. This is believed to be a result of the laboratory scale conditions and should be overcome when scaling up to ensure that all of the fibres are in contact with solutions. The images show the silver nanoparticles evenly distributed on the exterior and interior surfaces (in the pores) of the fibres.

Batches of fibres prepared according to the method above were found to have a silver content of 10.78, 10.33, 10.68, and 11.07% (measured by ICP-OES). Although the silver content was lower than for fibres made using method 1.1a, visible inspection suggested a higher proportion of silver nanoparticles as compared to silver oxide, than the fibres made in method 1.1a.

The pH of the fibres was measured by placing 0.5g fibre in 50 ml deionized water and the initial physical and mechanical properties of the fibres were measured. The results are shown in Table 1d.

**Table 1d**

| | **Initial Tensile Properties** | | | | **pH** |
|---|---|---|---|---|---|
| | **Linear Density, dtex** | **Break Load, cN** | **Breaking Tenacity, cN/tex** | **Strain at Break %** | |
| **Cellulose impregnated with silver nanoparticles (method 1.1b)** | 1.42 | 2.56 | 18.01 | 4.92 | 6.01 |

### 1.6 Release of metal from the hydroxide/carbonate cellulose fibres (method 1.1b)

0.5g of cellulose fibre impregnated with silver nanoparticles, made as described above, was placed into 50 ml deionized water and incubated at 37°C. Each subsequent day, 2 ml of the liquid was removed for analysis by UV-Vis. The liquid removed was replaced with fresh deionized water. The silver content of the liquid measured each day is shown in Table 2b.

**Table 2b**

| **Time (days)** | **Silver content (ppm)** |
|---|---|
| 1 | 34.5 |
| 2 | 35.0 |
| 3 | 36.4 |
| 4 | 39.2 |
| 5 | 35.4 |

The fibres are capable of providing a sustained release of silver over a period of time. This property is particular advantageous for wound dressings.

### Example 2: Production of a material comprising cellulose fibres impregnated with metal nanoparticles

### 2.1 Process for producing a non-woven fabric using cellulose fibres impregnated with metal nanoparticles

1. The cellulose fibres impregnated with metal nanoparticles are cut to short lengths of between 50 mm and 100 mm.
2. Hydrophilic, absorbent and/or gelling fibres are cut to the same length as the silver containing cellulose fibres.
3. The two types of fibres are then blended in the desired ratio using any technique known in the art for blending fibres. Preferably, the blended fibres are prepared using the carding technique whereby the fibres are opened, oriented and intimately blended. The carding is carried out as many times as necessary to give the most homogeneous distribution of silver as possible, whilst retaining fibre integrity and fabric strength. This ultimately aids release of metal from the final dressing and ensures even uptake of fluid from the wound across the whole wound contact area.
4. Once the blended fibres have been carded, they are cross folded to form a web of non-woven fibres which, at this stage, can be used to form a wound dressing. More preferred though, is subsequent needle punching of the web, which entangles the fibres together, providing rigidity, integrity and strength to the web.
5. The web can then be cut into appropriately sized pieces to be used as wound dressings e.g. 10cm x 10cm.

### Example 3: Absorbent materials comprising cellulose fibres impregnated with metal nanoparticles

### 3.1 Dressing compositions

Cellulose fibres impregnated with silver nanoparticles were prepared as described in Example 1. The fibres were then blended with other fibres and formed into wound dressings, using the method described in Example 2, to provide Dressings A, B and C. The composition of Dressings A and B is shown in Table 2. The composition of Dressing C is shown in Table 2b.

**Table 2a**

| **Fibre** | **Relative Amounts (% w/w)** | |
|---|---|---|
| | **Dressing A** | **Dressing B** |
| Silver Nanoparticle impregnated with Cellulose Fibre (20% w/w silver) via method 1.1 | 10 | 10 |
| Cellulose Fibre (e.g. Tencel™) | 10 | 10 |
| Cellulose Ethyl Sulphonate Fibre | 80 | - |
| Calcium Alginate Fibre | - | 80 |

The final silver content of both dressings was approximately 2% w/w.

**Table 2b**

| **Fibre** | **Relative Amounts (% w/w)** |
|---|---|
| | **Dressing C** |
| Silver Nanoparticle impregnated with Cellulose Fibre (20% w/w silver) via method 1.2 | 5.0 |
| Cellulose Fibre (e.g. Tencel™) | 25.0 |
| Carboxymethyl Cellulose Fibre | 70.0 |

The silver content of dressing C was approximately 0.5% w/w.

The formulated dressings were pale grey/silver in colour with a highly uniform level of colouration. They were found to be more aesthetically pleasing compared to some of the more highly coloured products available on the market currently.

### 3.2 Dressing A: Physico-Mechanical Testing

The typical physico-mechanical properties of Dressing A were measured using standard techniques, and compared to those of comparative Dressing D, which is a standard CES-based dressing (comprising CES and TENCEL (RTM)).

As shown in Table 3, Dressing A has improved tensile strength in the machine (MD) and cross (XD) directions, when both wet and dry, compared to the Comparative Dressing D.

**Table 3**

| **Laboratory Test** | **Comparative Dressing D** | **Dressing A** |
|---|---|---|
| Silver Content (ICP, % w/w) | NA | 1.92 (1.89-1.96) |
| Basis Weight (gm⁻²) | 132 | 128 |
| Absorbency (g/100cm²) | 23.1 | 22.4 |
| Retention (g/100cm²) | 12.7 | 11.6 |
| Lateral Wicking MD (cm) | - | 2.09 |
| Lateral Wicking XD (cm) | - | 2.07 |
| Dry Tensile Strength MD (Kgf.cm) | 0.160 (equivalent to 1.57-.N.cm) | 0.642 (equivalent to 6.30 N.cm |
| Dry Tensile Strength XD (Kgf.cm) | 0.410 (equivalent to 4.02 N.cm) | 1.912 (equivalent to 18.75 N.cm) |
| Wet Tensile Strength MD (Kgf.cm) | 0.027 (equivalent to 0.26 N.cm) | 0.086 0.84 (equivalent to N.cm) |
| Wet Tensile Strength XD (Kgf.cm) | 0.037 0.36 (equivalent to N.cm) | 0.191 1.87 (equivalent to N.cm) |

### 3.3 Dressing A: Silver release Profile

The release of silver nanoparticles from Dressing A was examined. 0.5g of wound dressing was placed into 25 ml of either deionised water or 0.9% sodium chloride solution and stored at 37°C. At regular intervals, a small sample of the liquid was taken out and examined using ultraviolet spectrophotometry (UV-vis), utilizing the phenomenon of "surface plasmon resonance" exhibited by the metallic nanoparticles. The portion of liquid that was taken away was replaced with fresh deionised water or sodium chloride solution. The silver contents were monitored over 4 days.

The spectrophotometer was calibrated using commercially available silver nanoparticles with an average particle size of 20 nm. These nanoparticles exhibit a surface plasmon resonance at 400-405 nm. The spectrum in Figure 1 shows the characteristic SPR peak used in this study.

Dressing A showed the following silver nanoparticle release characteristics; day 1 - 4.8 ppm; day 2 - 5.7 ppm; day 3 - 7.8 ppm; day 4 - 6.2 ppm. Dressing A was therefore found to be capable of providing a low, sustained release of silver. Dressing A is therefore advantageous compared to some known dressings (containing / releasing ionic silver) which show very fast release of silver on day 1, followed by rapid tailing off on subsequent days.

### 3.4 Dressing A: Antimicrobial testing - agar plates

The antimicrobial properties of Dressing A were assessed. Agar plates were inoculated with *Pseudomonas aeruginosa.* Four pieces of the dressing (or controls) of identical size and shape were then placed on the agar plates and incubated for 48 hours at 37°C. Plain gauze was used as the positive control. Fabric soaked in acetic acid was used as the negative control.

As expected, significant bacterial growth was observed on the agar plate containing the positive control. The negative control resulted in the total eradication of bacteria. Surprisingly, Dressing A also caused the total eradication of bacteria on the plate. Dressing A is therefore an effective antimicrobial material.

### 3.5 Dressing A: Antimicrobial testing - milk

The dressing was placed in 25g milk, in an appropriate weight to simulate a silver concentration of 100 ppm based on the weight of the milk (assuming 100% release from the fabric. The milk sample was sealed in a bottle and incubated at 37°C for 7 days. The results are shown in Table 4 below.

**Table 4**

| **Fabric Sample** | **Silver (% w/w)** | **Antimicrobial Performance @ 37°C** |
|---|---|---|
| Blank Sample - No Fabric | NA | PS, O (very strong malodour), GAS |
| | | Observed after 2 days |
| Comparative Example D Standard Cellulose Ethyl Sulphonate Based | NA | PS, O (very strong malodour), GAS |
| | | Observed after 2 days |
| Dressing A | 1.92 | NC |
| | | Observed after 7 days |

### Key

- PS: *Phase separation of milk*
- O: *Odour present*
- *GAS*: *Pressure build up in test vessel*
- *NC*: *No change*

Dressing A therefore shows significant antimicrobial properties by preventing the spoiling of milk.

### 3.6 Dressing B: Physico-Mechanical Testing

Table 5 shows the typical physico-mechanical properties of Dressing B as compared to a comparative Dressing E, which is a standard alginate-based dressing.

**Table 5**

| **Laboratory Test** | **Comparative Dressing E** | **Dressing B** |
|---|---|---|
| Silver Content (ICP, % w/w) | NA | 2.15 |
| Basis Weight (gm⁻²) | 127 | 125 |
| Absorbency (g/100cm²) | 19.7 | 20.7 |
| Retention (g/100cm²) | 7.6 | 5.8 |
| Dry Tensile Strength MD (Kgf.cm) | 0.255 (equivalent to 2.50 N.cm) | 0.193 (equivalent to 1.89 N.cm) |
| Dry Tensile Strength XD (Kgf.cm) | 0.591 (equivalent to 5.80 N.cm) | 0.537 (equivalent to 5.27 N.cm) |
| Wet Tensile Strength MD (Kgf.cm) | 0.449 (equivalent to 4.40 N.cm) | 0.465 (equivalent to 4.56 N.cm) |
| Wet Tensile Strength XD (Kgf.cm) | 0.938 (equivalent to 9.20 com) | 0.863 (equivalent to 8.46 N.cm) |

As shown in Table 5, Dressing B has similar tensile strength and improved absorbency compared to the Comparative Dressing E.

### 3.7 Dressing B: Antimicrobial testing - milk

The experiment was carried out as for Dressing A (see Example 3.5 above). The results are shown in Table 6 below.

**Table 6**

| **Fabric Sample** | **Silver (% w/w)** | **Antimicrobial Performance @ 37°C** |
|---|---|---|
| Blank Sample - No Fabric | NA | PS, O (very strong malodour), GAS |
| | | Observed after 2 days |
| Comparative Dressing E | NA | NC |
| | | Observed after 7 days |
| Dressing B | 1.92 | NC |
| | | Observed after 7 days |

Dressing B therefore also shows significant antimicrobial properties by preventing the spoiling of milk.

### 3.8 Dressing C - Physico-mechanical Testing

Table 7 shows the typical physico-mechanical properties of Dressing C as compared to comparative dressing F, a CMC based dressing

**Table 7**

| **Laboratory Test** | **Comparative Dressing F** | **Dressing B** |
|---|---|---|
| Silver Content (ICP, % w/w) | NA | 0.553 |
| Basis Weight (gm⁻²) | 126 | 128 |
| Absorbency (g/100cm²) | 25.1 | 26.2 |
| Retention (g/100cm²) | 10.6 | 12.4 |
| Dry Tensile Strength MD (Kgf.cm) | 0.973 (equivalent to 9.54 N.cm) | 1.010 (equivalent to 9.90 N.cm) |
| Dry Tensile Strength XD (Kgf.cm) | 2.411 (equivalent to 23.64 N.cm) | 2.787 (equivalent to 27.33 N.cm) |
| Wet Tensile Strength MD (Kgf.cm) | 0.167 (equivalent to 1.64 N.cm) | 0.182 (equivalent to 1.78 N.cm) |
| Wet Tensile Strength XD (Kgf.)cm | 0.286 (equivalent to 2.80 N.cm) | 0.421 (equivalent to 4.13 N.cm) |

As shown in Table 7, Dressing C has similar absorbency and retention properties to the comparative, non silver containing Dressing F, whilst showing improved wet tensile strength.

### 3.9 Dressing C - Antimicrobial Testing

More formal antimicrobial testing was performed with Dressing C, using the standard test method BS EN 16756 (in development) and the methods according to Gallant-Behm et al and Thomas and McCubbin, with repeat inoculation and enumeration of Staphylococcus aureus (ATCC6538) and Pseudomonas aeruginosa (ATCC9027) every 24 hours for seven days.

Gallant-Behm C.L. et al., Comparison of in vitro disc diffusion and time kill assays for the evaluation of antimicrobial wound dressing efficiency Wound Repair and Regeneration 2005 13; 4. 412-417*.*

Thomas and McCubbin, A comparison of the antimicrobial effects of four silver-containing dressings on three organisms. J of Wound Care 2003; 12; 3; 101-107*.*

Total kill of the bacteria was observed within 24 hours with both bacteria i.e. no viable, living bacteria were recovered at any time point during the seven day study. This proves that Dressing C is an effective antimicrobial product.

## Claims

1. A method of producing cellulose fibres impregnated with metal nanoparticles, the method comprising:
swelling cellulose fibres in an aqueous alkali solution;
removing the swollen cellulose fibres from the aqueous alkali solution;
mixing the swollen cellulose fibres with an aqueous solution of a metal salt and a polymer solution so as to impregnate the fibres with metal nanoparticles; and
removing the swollen cellulose fibres impregnated with metal nanoparticles from the solution,
wherein the aqueous alkali solution comprises Group I hydroxide and Group I carbonate and/or bicarbonate.

2. The method of claim 1, wherein the metal is silver, copper, zinc, selenium, gold, cobalt, nickel, zirconium, molybdenum, gallium or iron, or any combination thereof.

3. The method of any one of the preceding claims, further comprising incubating the cellulose fibres in the aqueous alkali solution at a temperature of from 20 °C to 120 °C, and/or incubating the mixture of the aqueous solution of metal salt, the polymer solution and the swollen cellulose fibres at a temperature of from 20 °C to 120 °C.

4. The method of any one of the preceding claims, further comprising washing the swollen cellulose fibres after their removal from the aqueous alkali solution, and prior to mixing the swollen cellulose fibres with the aqueous solution of a metal salt and the polymer solution.

5. The method of any one of the preceding claims, wherein the polymer is a polyamide, a polyimide, polyethyleneimine, polyvinylalcohol, pectin, albumin, gelatin, carrageenan, gum, cellulose or a derivative thereof, poly (N-vinylpyrrolidone), poly (N-vinylcaprolactam), or mixtures thereof.

6. The method of any one of the preceding claims, further comprising the step of washing the swollen cellulose fibres impregnated with metal nanoparticles after removal from the solution, optionally wherein the fibres are washed with an organic solvent to shrink the fibres.

7. Cellulose fibres impregnated with metal nanoparticles producible by the method of any one of the preceding claims.

8. The cellulose fibres of claim 7 impregnated with metal nanoparticles at a concentration of more than 1.5 w/w.

9. The cellulose fibres of claim 7 or 8, wherein the average particle size of the metal nanoparticles is from 5 to 50 nm.

10. The cellulose fibres of any one of claims 7 to 9, further impregnated with a metal oxide and/or a metal carbonate and, optionally, a polymer.

11. An absorbent material comprising a blend of the cellulose fibres according to any one of claims 7 to 10 with at least one other type of fibre, optionally wherein the other type of fibre is:
a gelling fibre based on alginate, modified cellulose, modified chitosan, guar gum, carrageenan, pectin, starch, polyacrylates or copolymers thereof, polyethyleneoxides or polyacrylamides, or mixtures thereof; and/or
a non-gelling fibre based on polyester, polyethylene, polypropylene, polyamide, cellulose, thermoplastic bicomponent fibres, glass fibres, or mixtures thereof.

12. The absorbent material claim 11, comprising more than 0.2% w/w metal, based on the total weight of the blended fibres.

13. An absorbent article comprising the absorbent material of claim 12.

## Patentansprüche

1. Verfahren für die Herstellung von Cellulosefasern, die mit Metallnanoteilchen imprägniert sind, wobei das Verfahren Folgendes umfasst:
Aufquellenlassen von Cellulosefasern in einer wässrigen Alkalilösung;
Entfernen der aufgequollenen Cellulosefasern aus der wässrigen Alkalilösung;
Mischen der aufgequollenen Cellulosefasern mit einer wässrigen Lösung eines Metallsalzes und einer Polymer Lösung, um die Fasern mit Metallnanoteilchen zu imprägnieren; und
Entfernen der aufgequollenen Cellulosefasern, die mit Metallnanoteilchen imprägniert sind, aus der Lösung,
wobei die wässrige Alkalilösung Gruppe I-Hydroxid und Gruppe I-Carbonat und/oder -Bicarbonat umfasst.

2. Verfahren nach Anspruch 1, wobei das Metall Silber, Kupfer, Zink, Selen, Gold, Kobalt, Nickel, Zirkonium, Molybdän, Gallium oder Eisen oder irgendeine Kombination davon ist.

3. Verfahren nach irgendeinem der vorhergehenden Ansprüche, ferner das Inkubieren der Cellulosefasern in der wässrigen Alkalilösung bei einer Temperatur von 20°C bis 120°C und/oder das Inkubieren der Mischung der wässrigen Lösung von Metallsalz, der Polymerlösung und der aufgequollenen Cellulosefasern bei einer Temperatur von 20°C bis 120°C umfassend.

4. Verfahren nach irgendeinem der vorhergehenden Ansprüche, ferner das Waschen der aufgequollenen Cellulosefasern nach ihrer Entfernung aus der wässrigen Alkalilösung und vor Mischen der aufgequollenen Cellulosefasern mit der wässrigen Lösung eines Metallsalzes und der Polymerlösung umfassend.

5. Verfahren nach irgendeinem der vorhergehenden Ansprüche, wobei das Polymer ein Polyamid, ein Polyimid, Polyethylenimid, Polyvinylalkohol, Pectin, Albumin, Gelatine, Carrageen, Gummi, Cellulose oder ein Derivat davon, Poly(N-Vinylpyrrolidon), Poly(N-Vinylcaprolactam) oder Mischungen davon ist.

6. Verfahren nach irgendeinem der vorhergehenden Ansprüche, ferner den Schritt des Waschens der aufgequollenen Cellulosefasern, die mit Metallnanoteilchen imprägniert sind, nach Entfernen aus der Lösung umfassend, wobei wahlweise die Fasern mit einem organischen Lösungsmittel gewaschen werden, um die Fasern zu schrumpfen.

7. Cellulosefasern, die mit Metallnanoteilchen imprägniert sind, die durch das Verfahren nach irgendeinem der vorhergehenden Ansprüche herstellbar sind.

8. Cellulosefasern nach Anspruch 7, die mit Metallnanoteilchen in einer Konzentration von mehr als 1,5 Gew./Gew. imprägniert sind.

9. Cellulosefasern nach Anspruch 7 oder 8, wobei die durchschnittliche Teilchengröße der Metallnanoteilchen 5 bis 50 nm beträgt.

10. Cellulosefasern nach irgendeinem der Ansprüche 7 bis 9, ferner mit einem Metalloxid und/oder Metallcarbonat und wahlweise einem Polymer imprägniert.

11. Absorptionsfähiges Material umfassend eine Mischung der Cellulosefasern nach irgendeinem der Ansprüche 7 bis 10 mit mindestens einem anderen Typ Faser, wobei wahlweise der andere Typ Faser Folgendes ist:
eine gelbildende Faser auf der Basis von Alginat, modifizierter Cellulose, modifiziertem Chitosan, Guargummi, Carrageen, Pectin, Stärke, Polyacrylaten, oder Copolymeren davon, Polyethylenoxiden oder Polyacrylamiden oder Mischungen davon und/oder
eine nichtgelbildende Faser auf der Basis von Polyester, Polyethylen, Polypropylen, Polyamid, Cellulose, thermoplastischen Bikomponentenfasern, Glasfasern oder Mischungen davon.

12. Absorptionsfähiges Material nach Anspruch 11, umfassend mehr als 0,2 Gew./Gew.-% Metall, auf das Gesamtgewicht der gemischten Fasern bezogen.

13. Absorptionsfähiger Artikel umfassend das absorptionsfähige Material nach Anspruch 12.

## Revendications

1. Procédé de production de fibres de cellulose imprégnées de nanoparticules métalliques, le procédé comprenant:
le gonflement des fibres de cellulose dans une solution alcaline aqueuse;
le retrait des fibres de cellulose gonflées de la solution alcaline aqueuse;
le mélange des fibres de cellulose gonflées avec une solution aqueuse d'un sel métallique et une solution polymère afin d'imprégner les fibres avec les nanoparticules métalliques; et
le retrait des fibres de cellulose gonflées imprégnées des nanoparticules métalliques de la solution,
la solution alcaline aqueuse comprenant un hydroxyde du Groupe I et un carbonate et/ou bicarbonate du Groupe I.

2. Procédé selon la revendication 1, le métal étant l'argent, le cuivre, le zinc, le sélénium, l'or, le cobalt, le nickel, le zirconium, le molybdène, le gallium ou le fer, ou n'importe quelle combinaison de ceux-ci.

3. Procédé selon l'une quelconque des revendications précédentes, comprenant en outre l'incubation des fibres de cellulose dans la solution alcaline aqueuse à une température de 20°C à 120°C, et/ou l'incubation du mélange de la solution aqueuse de sel métallique, de la solution polymère et des fibres de cellulose gonflées à une température de 20°C à 120°C.

4. Procédé selon l'une quelconque des revendications précédentes, comprenant en outre le lavage des fibres de cellulose gonflées après leur retrait de la solution alcaline aqueuse, et avant le mélange des fibres de cellulose gonflées avec la solution aqueuse d'un sel métallique et de la solution polymère.

5. Procédé selon l'une quelconque des revendications précédentes, le polymère étant un polyamide, un polyimide, un polyéthylèneimine, un poly(alcool de vinyle), la pectine, l'albumine, la gélatine, la carraghénane, la gomme, la cellulose ou un dérivé de celle-ci, la poly(*N*-vinylpyrrolidone), le poly(*N*-vinylcaprolactame), ou leurs mélanges.

6. Procédé selon l'une quelconque des revendications précédentes, comprenant en outre l'étape de lavage des fibres de cellulose gonflées imprégnées de nanoparticules métalliques après le retrait de la solution, optionnellement les fibres étant lavées avec un solvant organique pour rétrécir les fibres.

7. Fibres de cellulose imprégnées de nanoparticules métalliques pouvant être produites selon le procédé selon l'une quelconque des revendications précédentes.

8. Fibres de cellulose selon la revendication 7 imprégnées de nanoparticules métalliques à une concentration supérieure à 1,5 pds/pds.

9. Fibres de cellulose selon la revendication 7 ou 8, la taille moyenne de particule des nanoparticules métalliques étant de 5 à 50 nm.

10. Fibres de cellulose selon l'une quelconque des revendications 7 à 9, imprégnées en outre d'un oxyde métallique et/ou d'un carbonate métallique et, optionnellement, d'un polymère.

11. Matériau absorbant comprenant un mélange des fibres de cellulose selon l'une quelconque des revendications 7 à 10 avec au moins un autre type de fibre, optionnellement l'autre type de fibre étant:
une fibre gélifiante à base d'alginate, de cellulose modifiée, de chitosane modifié, de gomme de guar, de carraghénane, de pectine, d'amidon, de polyacrylates ou leurs copolymères, de poly(oxydes d'éthylène) ou de polyacrylamides, ou leurs mélanges; et/ou
une fibre non gélifiante à base de polyester, de polyéthylène, de polypropylène, de polyamide, de cellulose, de fibres bicomposées thermoplastiques, de fibres de verre, ou leurs mélanges.

12. Matériau absorbant selon la revendication 11, comprenant plus de 0,2 % de métal en pds/pds, sur la base du poids total des fibres mélangées.

13. Article absorbant comprenant le matériau absorbant selon la revendication 12.
